# EUROPEAN PATENT APPLICATION

(11) **EP 4 001 917 A1**
(43) Date of publication of application: **25.05.2022**
(21) Application number: 20207251.8
(22) Date of filing: 12.11.2020
(51) Int. Cl.: G01N 33/50, A61K 38/55, G01N 33/574, G01N 33/68

(54) **INHIBITORS FOR USE IN TREATING LIVER DISORDERS**

(71) Applicant: Deutsches Krebsforschungszentrum Stiftung des Öffentlichen Rechts, 69120 Heidelberg (DE)
(72) Inventor: HEIKENWÄLDER, Mathias, 69120 Heidelberg (DE); LI, Xin, 69120 Heidelberg (DE); RAMADORI, Pierluigi, 69120 Heidelberg (DE); HALLER, Dirk, 69120 Heidelberg (DE)
(74) Representative: Krauss, Jan

(57) **Abstract**

The invention is based on the finding that endoplasmic reticulum (ER) stress signalling is associated with the pathogenesis of liver disorders such fatty liver, cirrhosis and hepatocellular carcinoma (HCC), and others. The invention identifies novel targets for liver disease therapy which are involved in ER stress signalling, and thereby pertains to compounds and compositions for medical uses, screening approaches to identify therapeutics as well as diagnostic approaches for the identification of disorders or the stratification of certain liver disease patient groups.

## Description

### FIELD OF THE INVENTION

The invention is based on the finding that endoplasmic reticulum (ER) stress signalling is associated with the pathogenesis of liver disorders such fatty liver, cirrhosis and hepatocellular carcinoma (HCC), and others. The invention identifies novel targets for liver disease therapy which are involved in ER stress signalling, and thereby pertains to compounds and compositions for medical uses, screening approaches to identify therapeutics as well as diagnostic approaches for the identification of disorders or the stratification of certain liver disease patient groups.

### DESCRIPTION

Changes in our lifestyle and diet have dramatically increased the incidence of obesity, overweight and metabolic syndrome. Overweight and obesity are not only a problem in the developed countries of the Western world. Meanwhile, children and adults in developing countries are also strongly affected as well (Anstee et al., 2019). The latest WHO report predicts that the number of obese diseases will double to triple over the next two decades (Stewart and Wild, 2014).

The liver, the body's most important metabolic organ, is severely affected by chronic hypercaloric intake, obesity, sedentary lifestyle and the resulting pathologies. The most common liver disease worldwide, non-alcohol fatty liver disease (NAFLD), is a clinical manifestation of overweight/obesity and metabolic syndrome. NAFLD is a chronic disease that can last up to several decades. NAFLD is characterized by predominant macrovesicular steatosis and metabolic deterioration of the liver, but not necessarily by obvious clinical symptoms. Currently, 90 million Americans and 40 million Europeans have NAFLD (Ringelhan et al., 2018; Anstee et al., 2019). The close link between obesity and cancer incidence is well known (Calle and Kaaks, 2004). In fact, a significant number of NAFLD patients develop non-alcoholic steatohepatitis (NASH) - a pathological from of fatty liver disease, which predisposes to fibrosis and hepatocellular carcinoma (HCC) (Anstee et al., 2019) - or liver dysfunction.

Hepatocellular carcinoma (HCC) is the most prevalent primary liver cancer, accounting for 80-90% of all cases, with an average survival in the first 5 years after diagnosis of ∼20%. Intrahepatic cholangiocarcinoma (iCC) and extrahepatic bile-duct carcinoma account for 6-15% of liver cancers, with a further reduced 5-year survival of ∼5%. Chronic hepatitis caused by different aetiologies (e.g. viral and non-viral) preceding HCC/iCC leads to an immunosuppressive, pro-tumorigenic environment, which does not allow the immune system to mount efficient anti-tumour responses.

The number of people with NAFLD and NASH is steadily increasing in the USA and Europe (Loomba et al., 2013; Ringelhan et al., 2018; Anstee et al., 2019). Therefore, obesity, steatosis and steatohepatitis, which are the causes of the increasing incidence of liver dysfunction and HCC, are the main causes of attention in Western countries (White et al., 2012).

Until now, chronic viral infections (e.g. hepatitis B and C viruses) have been the most widespread etiology leading to the development of liver dysfunction and HCC in industrialized countries. However, the number of patients with NAFLD is increasing on a daily basis - and thus NAFLD has become the most prevalent liver disease in developed and developing countries. Consequently, HCC is the cancer with the highest growth rate of patients in the United States and a similar trend is already observed in Europe (Anstee et al., 2019). At the same time, there are no efficient therapeutics to treat NASH and the treatment options for late-stage NASH-induced HCC are limited (Villanueva et al., 2014; Ringelhan et al., 2018; Anstee et al., 2019). This is mainly due to the fact that until recently the most important mechanisms triggering this chronic inflammatory/metabolic liver disease have not been understood. As a consequence, specific therapeutic treatments are urgently needed that do not cause side effects.

Chronic necro-inflammation and hepatitis is the main driver of liver cancer^{[1,2]}. The endoplasmic reticulum (ER) in eukaryotic cells is responsible for protein folding and cellular protein trafficking in the secretory pathway, as well as calcium storage and release. ER protein folding can be disrupted by environmental, physiological and pathologic factors, resulting in ER stress^{[3]}. A group of highly conserved signalling pathways of the unfolded protein response (UPR) contribute to a productive ER protein-folding milieu. UPR-activation and the failure to resolve ER-stress can contribute to cancer^{[4,5]}. It is well established that chronic hepatitis is the major risk factor for the development of cancer in patients with hepatitis^{[1]}; however, the contribution of specific UPR-programs toward hepatocyte homeostasis and thus the involvement in inflammation and tumorigenesis, remains unclear. The UPR-signal transducer and ER transmembrane protein ATF6 triggers transcriptional programs that increase ER-capacity, protein folding, and degradation to remove misfolded proteins. ATF6 deletion in mice proved that it is dispensable for embryonic and postnatal development, if not combined with ATP6β or ER co-chaperone p58IPK deficiency^{[6]}. Nevertheless, lack of ATF6 compromises the secretory pathway and impairs adaptation to acute and chronic ER stress^{[6]}. Remarkably, in acute stress-experiments it has been shown *in vitro* and *in vivo* that ATF6 activation is crucial for enabling cell survival^{[7]}.

However, how chronic UPR-stress affects homeostasis, modulates the tissue microenvironment and potentially contributes to fatty liver disease and cancer, by yet ill-defined mechanisms, has remained elusive. In the past we have found that activation of the mitochondrial and UPR-stress pathway (e.g. via HSP60) led to the formation on iCC-neoplasia^{[4]}.

Thus, it is an objection of the invention to provide a novel approach for the prevention, treatment and diagnosis of liver diseases such as NASH, NAFLD and HCC, as well as approaches for the identification of future therapeutics useful in tackling such disorders.

### BRIEF DESCRIPTION OF THE INVENTION

Generally, and by way of brief description, the main aspects of the present invention can be described as follows:

In **a first aspect,** the invention pertains to an inhibitor of endoplasmic reticulum (ER) stress signalling for use in the treatment or prevention of a liver disease in a subject, comprising administering the inhibitor to the subject and thereby reducing or inhibiting endoplasmic reticulum (ER) stress signalling in a cell of the subject.

In **a second aspect,** the invention pertains to a pharmaceutical composition for use in the treatment of a liver disease in a subject, comprising an inhibitor recited in any one of the preceding claims and a pharmaceutically acceptable carrier and/or excipient, wherein the treatment comprises an administration of the pharmaceutical composition to the subject.

In **a third aspect,** the invention pertains to a method for determining whether a subject has, or is at risk of developing, a liver disease, the method comprising the step of:
- detecting an applicable biomarker in a biological sample from said subject;
wherein the detection of the applicable biomarker in the sample indicates a phenotype or a risk of developing a phenotype that is associated with the development of the liver disease; and wherein the applicable biomarker is one selected from the group consisting of:
- AFT6, in particular the presence (or an amount) of or expression and/or activity of AFT6, preferably of nuclear AFT6;
- ER stress or ER stress signalling.

In **a fourth aspect,** the invention pertains to a method for identifying and/or characterizing a compound suitable for the treatment of a liver disease in a subject, the method comprising the steps of
- Providing a hepatocyte,
- Inducing in the hepatocyte ER stress or ER stress signalling,
- Contacting the cell of (ii) with a candidate compound,
wherein a reduced ER stress or ER stress signalling in the cell compared to a control indicates that the compound is suitable for the treatment of the liver disease.

In **a fifth aspect,** the invention pertains to a method for identifying and/or characterizing a compound suitable for a treatment of a liver disease in a subject, the method comprising the steps of
(a) bringing into contact a first cell expressing AFT6 and the candidate compound; and
(b) determining:
   (i) the expression, activity, function and/or stability of protein or mRNA of AFT6, in particular, of proteolytically cleaved and/or nuclear AFT6, in the first cell; and
   (ii) the ER stress signalling, or ER stress response in the first cell,
   wherein:
   (i) a reduced expression, activity function and/or stability of the AFT6, in said first cell contacted with the candidate compound compared to said first cell not contacted with said candidate compound; and
   (ii) the ER stress signalling, or ER stress response in the first cell contacted with the candidate compound compared to ER stress signalling, or ER stress response of the first cell not contacted with the candidate compound;
indicates that the candidate compound is a compound suitable for the treatment of the liver disease.

In **a sixth aspect,** the invention pertains to a kit, or use of a kit, for use in a method of for screening of the fourth or fifth aspect, the kit comprising a selection of compounds suspected to reduced ER stress or ER stress signalling, or reduce the expression, activity function and/or stability of the AFT6.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

In **a first aspect,** the invention pertains to an inhibitor of endoplasmic reticulum (ER) stress signalling for use in the treatment or prevention of a liver disease in a subject, comprising administering the inhibitor to the subject and thereby reducing or inhibiting endoplasmic reticulum (ER) stress signalling in a cell of the subject.

In a preferred embodiment of the present invention the inhibitor of ER stress signalling is preferably an inhibitor of activating transcription factor 6 alpha (ATF6) which inhibits or reduces the expression, stability, organelle translocation (e.g. from the Golgi into the endoplasmic reticulum) activation and/or function of AFT6 or nuclear AFT6 (nAFT6).

In context of the present invention it was surprisingly found that ER stress signalling, and in particular signalling via ATF6, is associated with the development of HCC in vivo. Further, the examples demonstrate a causal interrelationship between ATF6 function and HCC development supporting the use of ATF6 inhibition for both prevention and treatment of HCC.

The endoplasmic reticulum ("ER") serves a number of functions, including the facilitation of protein folding and the transport of synthesized proteins. The term "endoplasmic reticulum stress" ("ER stress") refers to an imbalance between the demand for the synthesis of proteins and the folding capacity of the ER to meet that demand. The unfolded protein response ("UPR") is activated in response to an accumulation of unfolded or misfolded proteins in the ER lumen. The UPR aims to restore normal function of the cell by halting protein translation and activate the signalling pathways that lead to increasing the production of molecular chaperones involved in protein folding. If these objectives are not achieved within a certain time period or the disruption is prolonged, the UPR aims to initiate programmed cell death (apoptosis).

As used herein, the term "Unfolded Protein Response" (UPR) or the "Unfolded Protein Response pathway" refers to an adaptive response to the accumulation of unfolded proteins in the ER and includes the transcriptional activation of genes encoding chaperones and folding catalysts and protein degrading complexes as well as translational attenuation to limit further accumulation of unfolded proteins. Both surface and secreted proteins are synthesized in the endoplasmic reticulum (ER) where they need to fold and assemble prior to being transported. Since the ER and the nucleus are located in separate compartments of the cell, the unfolded protein signal must be sensed in the lumen of the ER and transferred across the ER membrane and be received by the transcription machinery in the nucleus. The unfolded protein response (UPR) performs this function for the cell. Activation of the UPR can be caused by treatment of cells with reducing agents like DTT, by inhibitors of core glycosylation like tunicamycin or by Ca-ionophores that deplete the ER calcium stores. In mammals, the UPR signal cascade is mediated by three types of ER transmembrane proteins: the protein-kinase and site- specific endoribonuclease IRE-1; the eukaryotic translation initiation factor 2 kinase, PERK/PEK; and the transcriptional activator ATF6. If the UPR cannot adapt to the presence of unfolded proteins in the ER, an apoptotic response is initiated leading to the activation of JNK protein kinase and caspases 7, 12, and 3. The most proximal signal from the lumen of the ER is received by a transmembrane endoribonuclease and kinase called IRE-i. Following ER stress, IRE-1 initiates splicing of the XBP- 1 mRNA, the spliced version of which, activates the UPR.

The term "Activating transcription factor 6" or "ATF6" shall generally refer to an intracellular protein found in hepatocytes. ATF6 protein is also known as "Cyclic AMP-dependent transcription factor ATF-6 alpha" whereas the gene encoding the protein is referred to as *ATF6.* The protein occurs as a precursor of the transcription factor form (processed cyclic AMP-dependent transcription factor ATF-6 alpha), which is embedded in the endoplasmic reticulum membrane. Endoplasmic reticulum stress promotes processing of this form, releasing the transcription factor form that translocates into the nucleus, where it activates transcription of genes involved in the unfolded protein response (UPR). The protein of ATF6 is shown as amino acid sequence in SEQ ID NO: 1 and can be identified in the UniProt database with the accession P18850 (https://www.uniprot.org/uniprot/P18850; UniProt database version of November 12, 2020).

Furthermore, the term "ATF6" in context of the invention may refer to variants or fragments of human ATF6. Such variants or fragments may be orthologs, homologs or paralogs of human ATF6. Alternatively, such variants or fragments are proteins having an amino acid sequence that is at least 50, 60, 70 ,80 ,85, 90, 95, 96, 97, 98, 99, or 100% identical to the sequence of the ectodomain of human ATF6, SEQ ID NO: 1, preferably of the full-length protein of human ATF6 or the processed variant thereof. Fragments of ATF6 are preferably proteins comprising the amino acid sequence of the proteolytic cleaved ATF6 (nuclear ATF6), such soluble versions lacking any transmembrane or membrane associated domains.

In one embodiment of these aspects, the compound that is an inhibitor of ER stress signalling, or that is an inhibitor of ATF6/nATF6 is an inhibitor of the expression, function, activity and/or stability of ATF6 or of a nuclear ATF6, or of a variant thereof (such as described above). Such inhibitor of the expression, function, activity and/or stability of ATF6 or of a nuclear ATF6, or of a variant thereof is a compound that inhibits an of ATF6 or nATF6 cell biologic or biochemic functions (in particular in context of ER stress signalling) such as a nuclear translocation, or nATF6 binding to the genome and mediating transcriptional control of certain genes of the UFP.

One further example of an inhibitor of the invention is an inhibitor of the proteolytic cleavage of a membrane bound AFT6, such as a protease inhibitor, for example an inhibitor of Site-2 protease (S2P). Such S2P inhibitor or ATF6 inhibitor is selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bi-functional compound (such as a PROTAC or a HyT molecule); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

Included in the present disclosure are in particular any small molecular compounds known to exert inhibitory activity towards ER stress signalling and in particular towards the expression, function or stability of ATF6. Such compounds were described before, for example in any of the following international patent applications published as WO/2019/118785, WO/2019/236710, WO/2019/195810 and WO/2020/176428. All compounds disclosed in the aforementioned patent publications shall be included in the present disclosure by reference.

In one particular set of embodiments, the ATF6 inhibitor is a nucleic acid.

The terms "nucleic acid", "polynucleotide" and "oligonucleotide" are used interchangeably throughout and include DNA molecules (e.g., cDNA or genomic DNA), RNA molecules (e.g., mRNA), analogues of the DNA or RNA generated using nucleotide analogues (e.g., peptide nucleic acids and non-naturally occurring nucleotide analogues), and hybrids thereof. The nucleic acid molecule can be single-stranded or double-stranded.

In the case of ATF6 inhibitors being CRISPR/Cas9 constructs and/or guide RNA/DNAs (gRNA/gDNA) and/or tracrRNAs, the basic rules for the design of CRISPR/Cas9 mediated gene editing approaches are known to the skilled artisan and for example reviewed in Wiles MV et al (Mamm Genome 2015, 26:501) or in Savić N and Schwank G (Transl Res 2016, 168:15).

In particular embodiments, the ATF6 inhibitor may be an inhibitory nucleic acid molecule, such as antisense nucleotide molecule including a siRNA or shRNA molecule, for example as described in detail herein below.

In more particular of such embodiments, the inhibitory nucleic acid (such as siRNA or shRNA) can bind to, such as specifically bind to, a nucleic acid (such as mRNA) that encodes or regulates the expression, amount, function, activity or stability of: (i) ATF6; or (ii) a gene that controls the expression, amount, function and/or stability of ATF6 and, for example, thereby modulates the expression, amount function, activity and/or stability of ATF6.

An inhibitor of ATF6 that is a nucleic acid can be, for example, an anti-sense nucleotide molecule, an RNA, DNA or PNA molecule, or an aptamer molecule. An anti-sense nucleotide molecule can, by virtue of it comprising an anti-sense nucleotide sequence, bind to a target nucleic acid molecule (eg based on sequence complementarity) within a cell and modulate the level of expression (transcription and/or translation) of ATF6, or it may modulate expression of another gene that controls the expression, function and/or stability of ATF6. Similarly, an RNA molecule, such as a catalytic ribozyme, can bind to and alter the expression of the ATF6 gene, or it can bind to and alter the expression of other genes that control the expression, function and/or stability of ATF6, such as a kinase molecule, interacting protein, a transcription factor for or repressor protein of ATF6. An aptamer is a nucleic acid molecule that has a sequence that confers it an ability to form a three-dimensional structure capable of binding to a molecular target.

An inhibitor of ATF6 that is a nucleic acid can be, for example, can further be a double-stranded RNA molecule for use in RNA interference. RNA interference (RNAi) is a process of sequence-specific gene silencing by post-transcriptional RNA degradation or silencing (prevention of translation). RNAi is initiated by use of double-stranded RNA (dsRNA) that is homologous in sequence to the target gene to be silenced. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3'end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesised in vitro and introduced into a cell by methods known in the art.

As described above, a modulator of the invention that is an RNAi molecule (such as an siRNA) may bind to and directly inhibit or antagonise the expression of mRNA of ATF6. However, a modulator of the invention that is an RNAi molecule (such as an siRNA) may bind to and inhibit or antagonise the expression of mRNA of another gene that itself controls the expression (or function or stability) of ATF6.

The sequence identity of the antisense molecule according to the invention in order to target a ATF6 mRNA (or to target mRNA of a gene controlling expression, function and/or stability of ATF6), is with increasing preference at least 75%, at least 80%, at least 85%, at least 90%, at least 95%, at least 98%, at least 99% and 100% identity to a region of a sequence encoding the ATF6 protein, as disclosed herein. Preferably, the region of sequence identity between the target gene and the modulating antisense molecule is the region of the target gene corresponding to the location and length of the modulating antisense molecule. For example, such a sequence identity over a region of about 19 to 21bp of length corresponding to the modulating siRNA or shRNA molecule). Means and methods for determining sequence identity are known in the art. Preferably, the BLAST (Basic Local Alignment Search Tool) program is used for determining the sequence identity with regard to one or more ATF6 RNAs as known in the art. On the other hand, preferred antisense molecules such as siRNAs and shRNAs of the present invention are preferably chemically synthesised using appropriately protected ribonucleoside phosphoramidites and a conventional RNA synthesiser. Suppliers of RNA synthesis reagents include Proligo (Hamburg, Germany), Dharmacon Research (Lafayette, CO, USA), Pierce Chemical (part of Perbio Science, Rockford, IL (USA), Glen Research (Sterling, VA, USA), ChemGenes (Ashland, MA, USA), and Cruachem (Glasgow, UK).

The ability of antisense molecules, siRNA, and shRNA to potently, but reversibly, silence genes in vivo make these molecules particularly well suited for use in the pharmaceutical composition of the invention which will be also described herein below. Ways of administering siRNA to humans are described in De Fougerolles et al., Current Opinion in Pharmacology, 2008, 8:280-285. Such ways are also suitable for administering other small RNA molecules like shRNA. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer- based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules, siRNA, shRNA or miRNA. Further, viral delivery systems comprise influenza virus envelopes and virosomes.

The antisense molecules, siRNAs, shRNAs may comprise modified nucleotides such as locked nucleic acids (LNAs). The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. Such oligomers are synthesised chemically and are commercially available. The locked ribose conformation enhances base stacking and backbone pre-organisation. This significantly increases the hybridisation properties (melting temperature) of oligonucleotides. Particularly preferred example of siRNAs is GapmeR (LNA^{™} GapmeRs (Exiqon)). GapmeRs are potent antisense oligonucleotides used for highly efficient inhibition of ATF6 mRNA (or of mRNA of a gene controlling expression, function and/or stability of ATF6). GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus.

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

The term "treatment" in the present invention is meant to include therapy, e.g. therapeutic treatment, as well as prophylactic or suppressive measures for a disease (or disorder or condition). Thus, for example, successful administration of a ATF6 inhibitor prior to onset of the disease results in treatment of the disease. "Treatment" also encompasses administration of a ATF6 inhibitor after the appearance of the disease in order to ameliorate or eradicate the disease (or symptoms thereof). Administration of a ATF6 inhibitor after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

The compounds of the present invention, or pharmaceutically acceptable salts thereof, may also be administered simultaneously with, prior to, or after administration of one or more of the therapeutic agents described herein. Thus, the invention includes combinatorial treatments where two or more ATF6 inhibitors that are different are administered in combination to the subject in order to increase treatment efficiency. Such combination therapy may include administration of a single pharmaceutical dosage formulation which contains a compound of the present invention and one or more additional agents given below, as well as administration of the compound of the present invention and each of additional agent in its own separate pharmaceutical dosage formulation. For example, a compound of the present invention and a chemotherapeutic agent, where the agent is an inhibitor ATF6. Furthermore, in some particular embodiments, combinatorial treatments of the invention may include the use of other anti-cancer agents such as taxol (paclitaxel), taxotere, etoposide, cisplatin, vincristine, vinblastine, and the like, can be administered to the patient either together in a single oral dosage composition such as a tablet or capsule, or each agent administered in separate oral dosage formulations or via intravenous injection. Where separate dosage formulations are used, the compounds of the present invention and one or more additional agents can be administered at essentially the same time, i.e., concurrently, or at separately staggered times, i.e., sequentially; combination therapy is understood to include all these regimens.

The term "non-alcoholic fatty liver disease" or "NAFLD", as used herein, refers to a group of conditions having in common the accumulation of fat in the hepatocytes, NAFLD ranges from simple fatty liver (steatosis), to non-alcoholic steatohepatitis (NASH), to cirrhosis (irreversible, advanced scarring of the liver). The term "NAFLD" includes any stage or degree of progression of the disease.

The term "non-alcoholic steatohepatitis" or "NASH", as used herein, relates to a significant form of chronic liver disease characterized by inflammatory and fatty infiltration of the liver that is not associated with alcohol consumption.

The term "disorder or condition associated with NAFLD or NASH" in context of the invention shall be any disorder of condition which is pathologically directly affected or influenced by NAFLD or NASH. Preferably the term includes a disorder or condition developing from NAFLD or NASH, meaning that such disorder or condition is caused by a progressing, for example untreated, NAFLD or NASH. A preferred example is a liver cirrhosis or hepatocellular carcinoma (HCC).

As used herein the term "hepatocellular carcinoma" or "HCC" refers to the most common type of liver cancer, also called malignant hepatoma. HCC may have many different causes, but in some preferred embodiments of the present invention, the underlying cause of the liver disease is non-alcoholic fatty liver disease (NAFLD). NAFLD is the most common liver disorder in the Western industrialized countries. It is considered to be the hepatic manifestation of the metabolic syndrome. Thus, NAFLD tends to develop in people who are overweight or obese, and/or who have diabetes, high cholesterol or high triglycerides. For most people, NAFLD cause no signs and symptoms, and no complications. But in some people with NAFLD, the fat that accumulates in the liver can cause inflammation and scarring in the liver that is believed to result in fibrosis and cirrhosis. This more serious form of NAFLD is sometimes called non-alcoholic steatohepatitis (NASH). It is worth noting that metabolic syndrome and type 2 diabetes have been demonstrated to be independent risk factors of HCC - patients suffering from such risk factors are preferred patients to be treated in accordance of the invention because preferably the treatment also includes preventive treatments using the compounds and methods of the invention to avoid, or reduce the chance of, developing NAFLD/NASH and/or HCC.

A treatment in context of the invention includes a preventive treatment in order to reduce the chance of developing the disorder, for example in a patient suffering from a risk factor of the disease. Preferably however, the treatment of the invention is alleviation or a reduced progression of NASH and/or HCC, or its symptoms or complications. A subject being at risk of developing NASH, is for example, and preferably a diabetic patient, an obese patient, or a patient suffering from the metabolic syndrome or from another metabolic disorder.

Preferred in some embodiments is that the treatment of the invention is a reduced, stalled, or reversed progression of NAFLD/NASH into liver cirrhosis, preferably a reduced, stalled, or reversed progression of NASH into hepatocellular carcinoma (HCC). Further preferred is that the treatment is a prevention of HCC in a NASH-patient at risk to develop cirrhosis and/or HCC.

In some preferred embodiments of the invention, the subject to be treated does not have a condition selected from the following group consisting of alcoholic liver injury, drug-induced liver injury, chronic active hepatitis, hepatic steatosis and hepatocyte apoptosis, and preferably wherein the patient suffers from an inflamed fatty liver.

In preferred embodiments of the first aspect, the cell is a cell associated with the liver disease, and preferably is a hepatocyte.

Any of the herein disclosed inhibitors preferably reduce the nuclear concentration of AFT6, such as reduces the translocation of AFT6 to the nucleus in the cell, preferably a translocation of Golgi located AFT6 to the nucleus. Such translocation being in context of the invention one major function of ATF6.

In accordance with the present disclosure a liver disease is associated with any one or a combination of the following: fatty liver, hepatocyte hyperproliferation (liver cancer), expression of pro-inflammatory or immune-suppressive chemokines, presence of immune-suppressive cells such as T regulatory cells (TREG), proinflammatory immune cells such as monocytes and Myeloid Derived Suppressor Cells (MDSC).

Also, preventive treatments of the diseases disclosed herein shall be encompassed by the invention such that the inhibitor of the invention when administered to a subject not suffering from the liver diseases reduces the chances of development of the liver disease in the subject. In addition, the treatment may be performed in a patient at risk of developing NASH, such as a diabetic patient, an obese patient, or a patient suffering from the metabolic syndrome or from another metabolic disorder.

In **a second aspect,** the invention pertains to a pharmaceutical composition for use in the treatment of a liver disease in a subject, comprising an inhibitor recited in any one of the preceding claims and a pharmaceutically acceptable carrier and/or excipient, wherein the treatment comprises an administration of the pharmaceutical composition to the subject.

Pharmaceutical Compositions and Routes of Administration Pharmaceutical compositions of the present invention comprise administering an effective amount of one or more inhibitors that inhibit or down-regulate the S2P activity (and/or an additional agent) dissolved or dispersed in a pharmaceutically acceptable carrier to a subject.

The phrases "'pharmaceutical" or "pharmacologically acceptable" refers to molecular entities and compositions that do not produce an adverse, allergic or other untoward reaction when administered to an animal, such as, for example, a human, as appropriate. The preparation of a pharmaceutical composition that contains at least one ATF6 inhibitor or additional active ingredient will be known to those of skill in the art in light of the present disclosure, and as exemplified by Remington's Pharmaceutical Sciences, 18th Ed. Mack Printing Company, 1990, incorporated herein by reference. Moreover, for animal (e. g., human) administration, it will be understood that preparations should meet sterility, pyrogenicity, general safety and purity standards as required by FDA Office of Biological Standards.

As used herein, 2pharmaceutically acceptable carrier" includes any and all solvents, dispersion media, coatings, surfactants, antioxidants, preservatives (e. g. , antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed.

Mack Printing Company, 1990, pp. 1289-1329, incorporated herein by reference). Except insofar as any conventional carrier is incompatible with the active ingredient, its use in the therapeutic or pharmaceutical compositions is contemplated.

A pharmaceutical composition of the present invention may comprise different types of carriers depending on whether it is to be administered in solid, liquid or aerosol form, and whether it needs to be sterile for such routes of administration as injection. A pharmaceutical composition of the present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (e. g., aerosol inhalation), injection, infusion, continuous infusion, localized perfusion bathing target cells directly, via a catheter, via a lavage, in cremes, in lipid compositions (e. g. , liposomes), or by other method or any combination of the foregoing as would be known to one of ordinary skill in the art (see, for example, Remington's Pharmaceutical Sciences, 18th Ed. Mack Printing Company, 1990, incorporated herein by reference).

The actual dosage amount of a composition of the present invention administered to an subject can be determined by physical and physiological factors such as body weight, severity of condition, the type of disease being treated, previous or concurrent therapeutic interventions, idiopathy of the patient and on the route of administration. The number of doses and the period of time over which the dose may be given may vary. The practitioner responsible for administration will, in any event, determine the concentration of active ingredient (s) in a composition and appropriate dose (s), as well as the length of time for administration for the individual subject.

In certain embodiments, pharmaceutical compositions may comprise, for example, at least about o. 1% of an active compound. In other embodiments, the active compound may comprise between about 2% to about 75% of the weight of the unit, or between about 25% to about 60%, for example, and any range derivable therein. In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, etc., can be administered, based on the numbers described above.

In any case, the composition may comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

An ATF6 inhibitor (s) of the present invention may be formulated into a composition in a free base, neutral or salt form. Pharmaceutically acceptable salts, include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine.

In embodiments where the composition is in a liquid form, a carrier can be a solvent or dispersion medium comprising but not limited to, water, ethanol, polyol (e.g., glycerol, propylene glycol, liquid polyethylene glycol, etc), lipids (e.g., triglycerides, vegetable oils, liposomes) and combinations thereof. The proper fluidity can be maintained, for example, by the use of a coating, such as lecithin; by the maintenance of the required particle size by dispersion in carriers such as, for example liquid polyol or lipids; by the use of surfactants such as, for example hydroxypropylcellulose; or combinations thereof such methods. In many cases, it will be preferable to include isotonic agents, such as, for example, sugars, sodium chloride or combinations thereof.

In certain aspects of the invention, the ATF6 inhibitors are prepared for administration by such routes as oral ingestion. In these embodiments, the solid composition may comprise, for example, solutions, suspensions, emulsions, tablets, pills, capsules (e.g., hard or soft shelled gelatin capsules), sustained release formulations, buccal compositions, troches, elixirs, suspensions, syrups, wafers, or combinations thereof. Oral compositions may be incorporated directly with the food of the diet. Preferred carriers for oral administration comprise inert diluents, assimilable edible carriers or combinations thereof. In other aspects of the invention, the oral composition may be prepared as a syrup or elixir. A syrup or elixir, and may comprise, for example, at least one active agent, a sweetening agent, a preservative, a flavoring agent, a dye, a preservative, or combinations thereof.

In certain preferred embodiments an oral composition may comprise one or more binders, excipients, disintegration agents, lubricants, flavoring agents, and combinations thereof. In certain embodiments, a composition may comprise one or more of the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, etc. ; or combinations thereof the foregoing. When the dosage unit form is a capsule, it may contain, in addition to materials of the above type, carriers such as a liquid carrier. Various other materials may be present as coatings or to otherwise modify the physical form of the dosage unit. For instance, tablets, pills, or capsules may be coated with shellac, sugar or both.

Additional formulations which are suitable for other modes of administration include suppositories. Suppositories are solid dosage forms of various weights and shapes, usually medicated, for insertion into the rectum, vagina or urethra. After insertion, suppositories soften, melt or dissolve in the cavity fluids. In general, for suppositories, traditional carriers may include, for example, polyalkylene glycols, triglycerides or combinations thereof. In certain embodiments, suppositories may be formed from mixtures containing, for example, the active ingredient in the range of about 0.5% to about 10%, and preferably about 1% to about 2%.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

In particular embodiments, prolonged absorption of an injectable composition can be brought about by the use in the compositions of agents delaying absorption, such as, for example, aluminum monostearate, gelatin or combinations thereof.

In **a third aspect,** the invention pertains to a method for determining whether a subject has, or is at risk of developing, a liver disease, the method comprising the step of:
- detecting an applicable biomarker in a biological sample from said subject;
wherein the detection of the applicable biomarker in the sample indicates a phenotype or a risk of developing a phenotype that is associated with the development of the liver disease; and wherein the applicable biomarker is one selected from the group consisting of:
- AFT6, in particular the presence (or an amount) of or expression and/or activity of AFT6, preferably of nuclear AFT6;
- ER stress or ER stress signalling.

In context of the present invention a biological sample may comprise cells or tissue of the subject, or an extract of such cells or tissue, in particular where such cells are those (usually, typically; or in the case or a specific subject as suspected to be) involved with the liver disease (eg hepatocytes, or tumour cells such as cells of HCC).

In some embodiments of these detection, determination and/or diagnosis methods, the biological sample is a tissue sample from the subject, such as a sample of a liver tumour or a cancer from the subject. As described above, such tissue sample may be a biopsy sample of the tumour or a cancer such as a needle biopsy sample, or a tumour biopsy section or an archival sample thereof. Such a tissue sample may comprise living, dead or fixed cells, such as from the tumour or a cancer, and such cells may be suspected of expressing (e.g. aberrantly or localised) the applicable biomarker to be determined

Preferably a liver disease is a fatty liver, a non-alcoholic fatty liver disease (NAFLD), non-alcoholic steatohepatitis (NASH), liver cirrhosis or hepatocellular carcinoma (HCC)

Such detection, determination and/or diagnosis methods can be conducted as an in-vitro (eg ex-vivo) method, and can be, for example, practiced using the kit of the present invention (or components thereof). An in-vitro method may use, involve or be practised on immortalised cell lines (such as those replicated, cultured or indefinitely maintained outside of the body of an animal or human), or it may be use, involve or be practised in-vitro using cells (such as primary cells) directly or freshly obtained from the body of an animal of human (eg, practised as a so-called "ex-vivo" method).

In **a fourth aspect,** the invention pertains to a method for identifying and/or characterizing a compound suitable for the treatment of a liver disease in a subject, the method comprising the steps of
- Providing a hepatocyte,
- Inducing in the hepatocyte ER stress or ER stress signalling,
- Contacting the cell of (ii) with a candidate compound,
wherein a reduced ER stress or ER stress signalling in the cell compared to a control indicates that the compound is suitable for the treatment of the liver disease.

In **a fifth aspect,** the invention pertains to a method for identifying and/or characterizing a compound suitable for a treatment of a liver disease in a subject, the method comprising the steps of
(a) bringing into contact a first cell expressing AFT6 and the candidate compound; and
(b) determining:
   (i) the expression, activity, function and/or stability of protein or mRNA of AFT6, in particular, of proteolytically cleaved and/or nuclear AFT6, in the first cell; and
   (ii) the ER stress signalling, or ER stress response in the first cell,
   wherein:
   (i) a reduced expression, activity function and/or stability of the AFT6, in said first cell contacted with the candidate compound compared to said first cell not contacted with said candidate compound; and
   (ii) the ER stress signalling, or ER stress response in the first cell contacted with the candidate compound compared to ER stress signalling, or ER stress response of the first cell not contacted with the candidate compound;
indicates that the candidate compound is a compound suitable for the treatment of the liver disease.

The candidate compound used in the screening methods of the aforementioned aspects maybe one selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

Preferred examples of such candidate compounds are in particular any small molecular compounds known to exert inhibitory activity towards ER stress signalling and in particular towards the expression, function or stability of ATF6. Such compounds were described before, for example in any of the following international patent applications published as WO/2019/118785, WO/2019/236710, WO/2019/195810 and WO/2020/176428. All compounds disclosed in the aforementioned patent publications shall be included in the present disclosure by reference.

In certain embodiments of such screening aspects of the invention, the activity of the (eg protein or mRNA of) ATF6 in the first cell or cell free system may be determined directly (eg, by antibody detection of ATF6 protein or by PCR of ATF6 mRNA), or may be determined by determining the presence or an amount of cytoplasmic ATF6 or of a proteolytically cleaved, ATF6, preferably of nuclear ATF6, in the first cells or first cell free system, in particular in the cytoplasm of the first cell. For example, a reduction in cytoplasmic ATF6 and increase of nuclear ATF6 of such first cell would indicate an activity of ATF6 in such cell.

In a sixth aspect, the invention pertains to a kit, or use of a kit, for use in a method of for screening of the fourth or fifth aspect, the kit comprising a selection of compounds suspected to reduced ER stress or ER stress signalling, or reduce the expression, activity function and/or stability of the AFT6.

All references, patents, and publications cited herein are hereby incorporated by reference in their entirety.

### BRIEF DESCRIPTION OF THE FIGURES AND SEQUENCES

The figures show:

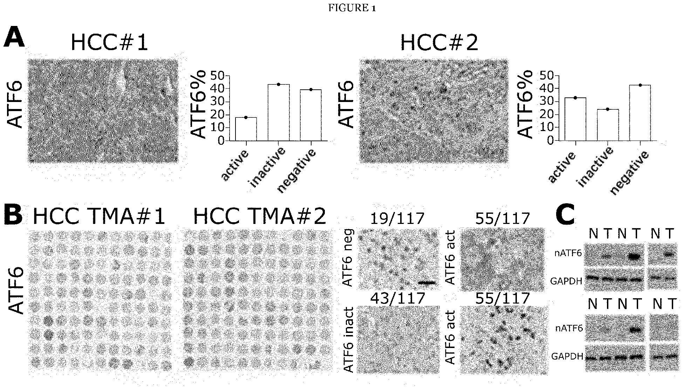

**Figure 1****:** shows ATF6, nATF6 expression and NK-cell phenotype in human liver cancer. (A) Representative immuno-histology staining and quantification of ATF6 expression and localization/activation in two HCC patients. Scale bar: 100µm (B) Analysis of distinct HCC tissue microarrays (TMA) corroborating the distribution of distinct nATF6 phenotype (one cohort is indicated with respective numbers). Scale bar: 25µm. (C) Western blot analysis of selected HCC patients confirming predominant nATF6 in tumour tissue (T) but not or only minor in non-tumour affected tissue; Statistics: *: p<0.05, ****: p<0.0001.

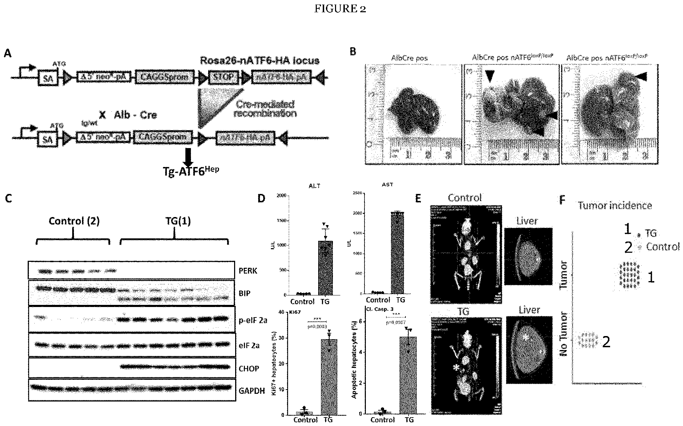

**Figure 2****:** shows Alb-Cre nATF6 mice exhibit an increased risk for liver cancer development. (A) Knock-in construct describing the Rosa26-nATF6-HA locus and activation of the latter upon Cre-mediated cleavage of the stop-cassette inform of the Alb-Cre nATF6 ORF. (B) Macroscopy of livers of Alb-Cre nATF6 mice and controls demonstrating tumorigenesis at 8 months of age. (C) Western blot analyses in tumour non-affected liver tissue demonstrate induction of ER-stress/UPR markers. (D) Serology (ALT, AST) and densitometric analysis of immunohistochemistry for positive hepatocytes in tumour non-affected tissue of Alb-Cre nATF6 and control mice. (E) PET-MRI identifies liver cancer in Alb-Cre nATF6 mice but not in controls (whole body and liver transection). (F) Tumour incidence of Alb-Cre nATF6 and control mice at 12 months of age.

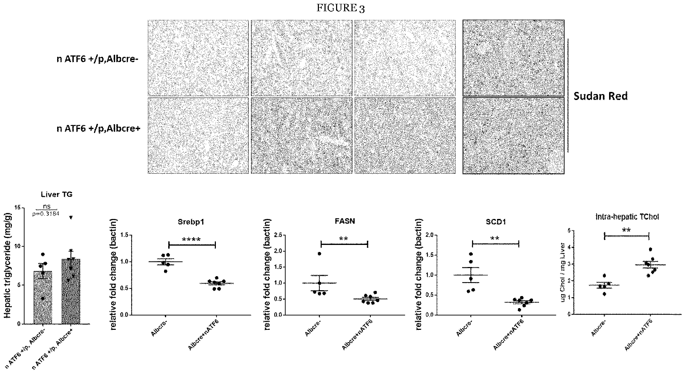

**Figure 3****:** shows Altered lipid metabolism in nATF6+/p, AlbCre+ mice. (upper figure) Lipid analysis for lipid droplets in transgenic and Wt mice (Sudan red). (lower figures) Real time PCR on hepatocyte metabolism genes, indicating that hepatocyte, lipid metabolism is shut down - consequently lipid droplets accumulate.

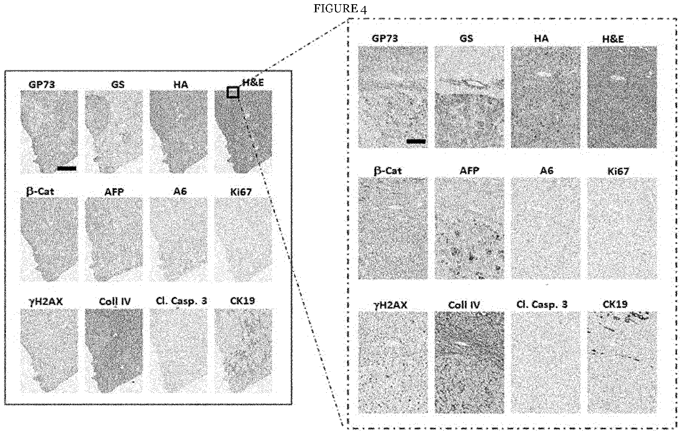

**Figure 4**: shows Tumours in Alb-Cre nATF6 mice qualify as HCC. (Left image) Macroscopic analyses of representative tumour nodules in Alb-Cre nATF6 mice, with several markers found in different human HCC subtypes. GP73 (Glycoprotein 73), GS (glutamine synthetase) are markers that can be found e.g. in viral-induced human HCC. HA: (influenza hemagglutinin-Tag) identifies the expression of the transgene. H&E: Hematoxilin/Eosin identifies cell-types, nuclear shape, cellular shape, proteinaceous deposits. β-Cat: β-catenin. AFP: alpha fetoprotein for hepatocyte regeneration, proliferation. A6: marker for bi-potent progenitor cell cells. Ki67: Marker for proliferation. γH2AX: marker for DNA damage and DNA damage response. Coll IV: Collagen IV, whose expression is usually lost in HCC. Cl. Casp.3: apoptosis marker. CK19: Marker for cholangiocytes. Scale bar: 350µm. (Right image) Inset of the depicted tumor-area of one tumour nodule, at the tumor-border. Scale bar: 350µm.

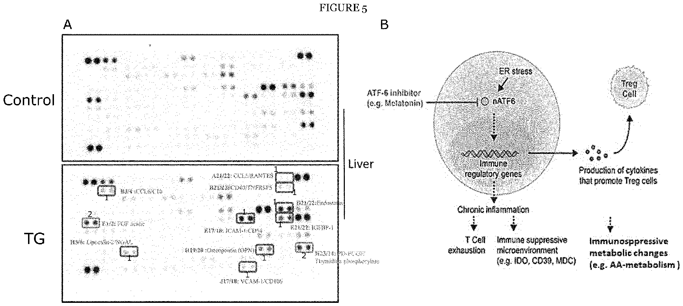

**Figure 5**: shows nATF6 induced immunological changes. (A) Cytokine/chemokine array of the liver of Alb-Cre negative versus Alb-Cre nATF6 mice, indicating upregulation of multiple immuno-regulatory cytokines/chemokines in the liver of nATF6 Alb-Cre positive mice. (B) Proposed mechanisms of action how nATF6 might modulate immune regulatory genes and thereby the TME in HCC towards an immune-suppressive phenotype.

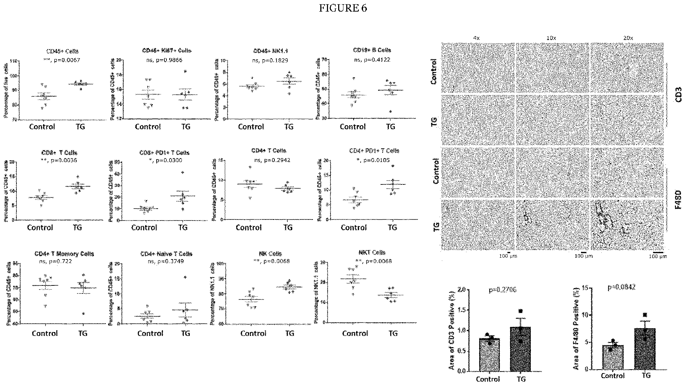

**Figure 6****:** shows Immunosuppressive Tregs, inflammatory monocytes and MDSCs are increased in livers of Alb-Cre nATF6 mice. (A) Flow-cytometry based quantification of different immune cell subsets in the liver of 6 or 8 months-old nATF6 Alb-Cre negative (green) versus nATF6 Alb-Cre positive (red) mice. (B) IHC-based quantification of CD3+ T cells and F480+ macrophages in the liver of 6 or 8 months-old Alb-Cre nATF6 (red) versus control (green) mice. Statistics: *: p<0.05, **: p<0.01.

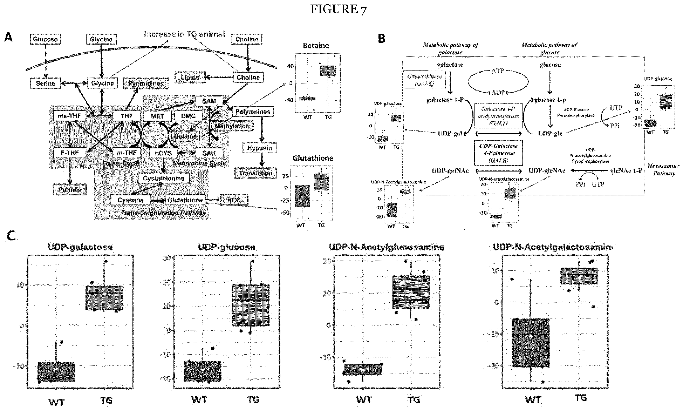

**Figure 7****:** shows a Metabolic analysis of liver homogenates of 8-week old Alb-Cre ATF6 transgenic mice by 1H NMR-metabolomics analysis. (A) Analysis of amino-acid metabolism reveals changes in betaine and gluthatione. (B) Alterations in Glucose metabolism (enlarged in C). Relative changes are displayed as fold change. All analyses displayed are statistically significant (p<0.05).

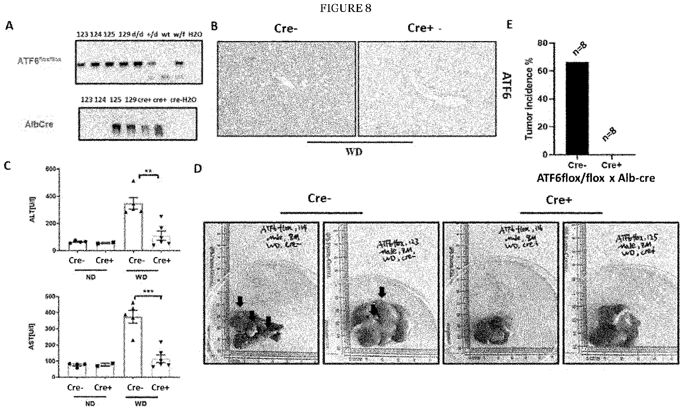

**Figure 8****:** shows a hepatocyte-specific inhibition of ATF6 is efficient, reduces NASH-induced liver damage and abrogates liver cancer development. (A) PCR of genomic DNA isolated from tail tissue for the identification of mice with hepatocyte-specific deletion of ATF6 in liver tissue. Numbers 123-129 depict individual mice. d/d: d= deleted. Controls from the original founder ATF6loxP/loxP mouse line. WT: wild-type for ATF6 from the intercrossed line (Alb-Cre). +/d: One allele ATF6 from the founder line (ATF6loxP/loxP), one allele from a wild-type mouse (C57BL/6J). w/f: floxed/wt allele for ATF6 from intercrossed mice (Alb-Cre). (B) Immunohistochemistry for ATF6 expression in livers of controls and hepatocyte-specific knockout. Only minor ATF6 expression is found in non-parenchymal cells in livers of WT or hepatocyte-specific deleted mice. (C) Reduced liver damage in a chronic model of NASH. WD: Western diet. (D) Macroscopy of liver with and without tumors. Liver tumor nodules are indicated by arrows. (E): Tumour incidence in WT or hepatocyte-specific deleted mice. Statistics: **: p<o.oi, ***: p<o.ooi.

The sequences show:
**SEQ ID NO: 1 shows the amino acid sequence of human ATF6**

10 20 30 40 50


60 70 80 90 100

110 120 130 140 150

160 170 180 190 200

210 220 230 240 250

260 270 280 290 300

310 320 330 340 350

360 370 380 390 400

410 420 430 440 450

460 470 480 490 500

510 520 530 540 550

560 570 580 590 600

610 620 630 640 650

660 670


### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: ATF6 expression and chronic ER-stress promote liver cancer

The initial studies of ATF6 expression in human chronic hepatitis (e.g. viral and non-viral) and liver cancer revealed two important findings: First, cleavage of ATF6 and subsequent activation of the ER-stress pathways by translocation of ATF6 to the nucleus (nATF6) is found in chronically inflamed tissue as well as in tumour tissue of HCC, CCC and mixed HCC/CCC patients, while being almost absent in healthy liver tissue. Expression and activation patterns of nATF6 in tumour tissue of HCC are heterogeneous suggesting potential treatment-stratification of the patients/tumours based on activated, cleaved nATF6 (Figure 1). The inventors hypothesized that an ER-stress/nATF6-axis that might contribute to immune-alterations leading to fatty liver disease, contribute to NASH and immune evasion, immunosuppression, both in preclinical mouse models as well as in human liver cancer.

Notably, in mouse models hepatocyte specific expression of nATF6 in Albumin producing hepatocytes (nATF6-HA Alb-Cre mice, further denoted as Alb-Cre nATF6) leads to increased liver damage (e.g. ALT, AST, Cleaved Casp.3), fatty liver disease (lipid droplets), hyper-proliferation of hepatocytes (Ki67) and signs of ER-stress, consequently leading to a strongly elevated risk for liver cancer development (Figure 2A-F and Figure 3). Liver cancer nodules displayed a heterogenous appearance, and different size and number (Figure 2B), and liver tumors consisted in majority of hepatocytes with nATF6 (>95%; Figure 4; revealed by influenza hemagglutinin Tag-staining). Notably, the observed ER stress in Alb-Cre nATF6 mice triggered multiple immuno-regulatory chemokines and cytokines in the liver compared to controls, known to be pro-inflammatory or immunosuppressive (Figure 4). These findings indicated that ATF6 expression and chronic ER-stress promote liver cancer with an immune-suppressive, pro-inflammatory tumor microenvironment (TME) supporting the concept of a functional link between the proto-oncogene nATF6 and immune escape in liver cancer (depicted in Figure 5B).

### Example 2: Alb-Cre nATF6 mice qualify as HCC model

It was then investigated whether tumours in Alb-Cre nATF6 mice would qualify as real HCC or iCC and thus the inventors performed detailed histological analyses of the tumours that were found in Alb-Cre nATF6 but not control mice (C57Bl/6J) at 10 months of age. Notably, comparing these tumours with human HCC the inventors could indeed demonstrate that murine tumours resemble characteristics found in human HCC, verifying Alb-Cre nATF6 mice as a valuable, preclinical mouse model of liver cancer. In seldom cases HCC/iCC mixed tumours in Alb-Cre nATF6 mice were observed, as well as multiple small and large tumour nodules indicative of intrahepatic metastasis. In line, a strong ductular response outside/nearby the tumour nodules was observed, indicative of chronic tissue damage (e.g. through ER stress) and a possible reason for the appearance of HCC/iCC mixed tumours (Figure 4).

### Example 3: Nuclear ATF6 triggers immune suppressive environment in HCC

Notably, besides elevated liver damage and increased hepatocyte proliferation and DNA damage (Figure 4), a remarkable change in the hepatic immune landscape of Alb-Cre nATF6 liver tissue (tumour non-affected) was observed. This was best characterized on protein level by changes in chemokine and cytokine expression (Figure 5A), already from 3 months of age onwards. The most striking changes included chemokines that affect intra-hepatic myeloid cell populations (e.g. CCL5, CCL2 - affecting homing of myeloid suppressor cells (MDSCs)), adhesion molecules V-CAM and I-CAM as well as molecules involved in immunosuppression (e.g. Lipocalin; involved in steroid triggered immune-suppression; IGFBP1).

Based on these data and data shown below that nATF6 function orchestrates the liver microenvironment towards an immunosuppressive status. Thus, chronic nATF6 expression eases tumour-formation in the absence of efficacious immune anti-tumour responses - not only shifting the inflammatory but also the metabolic liver environment (see Figure 7). Consequently, therapeutic or genetic suppression of nATF6 function might indeed allow to lift more effective anti-tumour immune responses. Moreover, it could even be possible to amplify the latter in combination with distinct immunotherapeutic treatments including anit-PD1-related immunotherapy but also the recently FDA-approved combination of atezolizumab (anti-PDLi) plus bevacizumab (anti-VEGF) (Figure 5B).

The data presented in Figure 5 have been further corroborated by flow cytometry analyses of intrahepatic immune cells from Alb-Cre nATF6 and control mice. Besides, the inventors performed a histological analysis of T-cells, myeloid cells and other cell types to get an idea on the local distribution of intrahepatic immune cells in the context of Alb-Cre nATF6 mice. The characterisation of immune cell infiltrates in the liver of in 6 or 8 months-old nATF6 Alb-Cre negative versus Alb-Cre nATF6 mice revealed an increase of CD8+PD1+ T cells, of immunosuppressive regulatory T-cells (Treg), NK and NKT cells as well as MDSCs (Figure 5A). There was a trend in the rise of total CD3+ cells and macrophages (F4/80+) as shown by immunohistochemistry (Figure 6B).

The results show that increased, chronic nATF6 expression is found in hepatocytes of chronic hepatitis and liver cancer patients, in several of those patients to a high degree within tumours. nATF6 expression leads to ER-stress, elevated DNA damage and causes profound changes of the hepatic cytokine profile. The latter alters the quality of immune cell infiltrates towards an increased intrahepatic accumulation of tolerogenic immune-cells in Alb-Cre nATF6 mice, finally leading to liver cancer. Aberrant, chronic nATF6 expression and alterations in hepatic immune-cell infiltrates are linked in HCC development in preclinical mouse models as well as human liver cancer.

### Example 4: Chronic nuclear ATF6 induced metabolic disturbances

Altered metabolism is a key feature of supporting an immune suppressive environment. Thus, it was addressed whether chronic activation of nATF6 in Alb-Cre nATF6 hepatocytes would induce metabolic disturbances supporting an immunosuppressive, pro-carcinogenic environment. Therefore, the inventors performed a 1H NMR-metabolomics analysis of mouse liver polar metabolites as a high throughput analysis on liver tissue of Alb-Cre ATF6 transgenic and control mice in a time course analysis. The initial data clearly show that indeed amino-acid metabolism as well as glucose metabolism was massively altered already at early time point in life (3 months of age) in livers of Alb-Cre ATF6 transgenic mice. Remarkably, at these time points no major macroscopic changes were found in liver tissue - suggesting these changes to be directly linked to nATF6 expression (Figure 7). Notably, first analyses reveal strong changes in betaine and glutathione, known to affect MDSC activity to effectively suppress CD8+ T-cell function^{[15]}. Moreover, a strong change in glucose metabolism was observed, supporting a pro-proliferative environment, which also alters adaptive and innate immune-cell behaviour.

### Example 5: Inhibition of ATF6 suppresses tumour development in vivo.

However, to corroborate that therapy-related or genetic inhibition of ATF6-function in hepatocytes could be (i) linked to the block of immunosuppressive effects in the context of liver cancer and (ii) could be thus used as a basis for strategies to intervene with an immunosuppressive TME, the inventors set out to knock-out ATF6 specifically in hepatocytes (Alb-Cre-ATF6^{loxP/loxP}). To do so, liver cancer was induced in a spontaneous, preclinical mouse model ^{[16]} (through a NASH diet; WD: Western diet), and it was tested whether suppression of ATF6 in hepatocytes would suppress tumour development over time. Notably, the data demonstrate that hepatocyte specific deletion of ATF6 was >than 90% efficient on mRNA level as well as very efficient on protein level (Figure 8). This led to reduced liver damage and abrogated NASH-induced liver cancer development (50% after 9 months of diet) in Alb-Cre-ATF6^{loxP/loxP}: In contrast to Alb-Cre- ATF6^{loxP/loxP} mice, which have developed in 4/8 mice tumours, 0/8 Alb-Cre- ATF6^{loxP/loxP} developed HCC.

This demonstrates that nATF6-related liver damage and cancer development is linked to direct and indirect changes of the liver TME causing immunosuppression through various mechanisms (e.g. NK, NKT-cell alteration, metabolic reprogramming, MDSC-attraction).

### REFERENCES

The references are:
[1] Ringelhan M, Pfister D, O'Connor T, Pikarsky E, Heikenwalder M. The immunology of hepatocellular carcinoma. Nature Immunology. 2018 Mar;19(3):222-232.
[2] Boege Y, Malehmir M, Healy ME, Bettermann K, Lorentzen A, Vucur M, Ahuja AK, Böhm F, Mertens JC, Shimizu Y, Frick L, Remouchamps C, Mutreja K, Kähne T, Sundaravinayagam D, Wolf MJ, Rehrauer H, Koppe C, Speicher T, Padrissa-Altés S, Maire R, Schattenberg JM, Jeong JS, Liu L, Zwirner S, Boger R, Hüser N, Davis RJ, Müllhaupt B, Moch H, Schulze-Bergkamen H, Clavien PA, Werner S, Borsig L, Luther SA, Jost PJ, Weinlich R, Unger K, Behrens A, Hillert L, Dillon C, Di Virgilio M, Wallach D, Dejardin E, Zender L, Naumann M, Walczak H, Green DR, Lopes M, Lavrik I, Luedde T, Heikenwalder M*, Weber A*. A Dual Role of Caspase-8 in Triggering and Sensing Proliferation-Associated DNA Damage, a Key Determinant of Liver Cancer Development. Cancer Cell. 2017 Sep 11;32(3):342-359.
[3] Karagöz GE, Acosta-Alvear D, Walter P. The Unfolded Protein Response: Detecting and Responding to Fluctuations in the Protein-Folding Capacity of the Endoplasmic Reticulum. Cold Spring Harb Perspect Biol. 2019 Sep 3;11(9):a033886.
[4] Berger E, Rath E, Yuan D, Waldschmitt N, Khaloian S, Allgäuer M, Staszewski O, Lobner EM, Schöttl T, Giesbertz P, Coleman OI, Prinz M, Weber A, Gerhard M, Klingenspor M, Janssen KP, Heikenwalder M, Haller D. Mitochondrial function controls intestinal epithelial sternness and proliferation. Nature Communications. 2016 Oct 27;7:13171.
[5] Yuan D, Huang S, Berger E, Liu L, Gross N, Heinzmann F, Ringelhan M, Connor TO, Stadler M, Meister M, Weber J, Öllinger R, Simonavicius N, Reisinger F, Hartmann D, Meyer R, Reich M, Seehawer M, Leone V, Höchst B, Wohlleber D, Jörs S, Prinz M, Spalding D, Protzer U, Luedde T, Terracciano L, Matter M, Longerich T, Knolle P, Ried T, Keitel V, Geisler F, Unger K, Cinnamon E, Pikarsky E, Hüser N, Davis RJ, Tschaharganeh DF, Rad R, Weber A, Zender L, Haller D*, Heikenwalder M*. Kupffer-cell derived TNF triggers cholangiocellular tumorigenesis through JNK due to chronic mitochondrial dysfunction and ROS. Cancer Cell. 2017, 31:771-789.
[6] Lee AH, Iwakoshi N, Glimcher L. XBP-1 regulates a subset of endoplasmic reticulum resident chaperone genes in the unfolded protein response Mol Cell Biol. 2003 Nov;23(21):7448-59.
[7] Papaioannou A, Higa A, Jégou G, Jouan F, Pineau R, Saas L, Avril T, Pluquet O, Chevet E. Alterations of EDEM1 functions enhance ATF6 pro-survival signalling. FEBS J. 2018, 285: 4146-4164.
[8] Alfei F, Kanev K, Hofmann M, Wu M, Ghoneim HE, Roelli P, Utzschneider DT, von Hoesslin M, Cullen JG, Fan Y, Eisenberg V, Wohlleber D, Steiger K, Merkler D, Delorenzi M, Knolle PA, Cohen CJ, Thimme R, Youngblood B, Zehn D. TOX reinforces the phenotype and longevity of exhausted T cells in chronic viral infection. Nature. 2019 Jul;571(7764):265-269
[9] Zheng C, Zheng L, Yoo JK, Guo H, Zhang Y, Guo X, Kang B, Hu R, Huang JY, Zhang Q, Liu Z, Dong M, Hu X, Ouyang W, Peng J, Zhang Z. Landscape of infiltrating T cells in liver revealed by single-cell sequencing. Cell. 2017, 169(7):1342-1356.
[10] Wang X, He Q, Shen H, Xia A, Tian W, Yu W, Sun B. TOX promotes the exhaustion of antitumor CD8+ T cells by preventing PDi degradation in hepatocellular carcinoma. J Hepatol. 2019 Oct;71(4):731-741.
[11] Khan O, Giles JR, McDonald S, Manne S, Ngiow SF, Patel KP, Werner MT, Huang AC, Alexander KA, Wu JE, Attanasio J, Yan P, George SM, Bengsch B, Staupe RP, Donahue G, Xu W, Amaravadi RK, Xu X, Karakousis GC, Mitchell TC, Schuchter LM, Kaye J, Berger SL, Wherry EJ. TOX transcriptionally and epigenetically programs CD8(+) T cell exhaustion. Nature. 2019, 571(7764):211-218.
[12] Scott AC, Dündar F, Zumbo P, Chandran SS, Klebanoff CA, Shakiba M, Trivedi P, Menocal L, Appleby H, Camara S, Zamarin D, Walther T, Snyder A, Femia MR, Comen EA, Wen HY, Hellmann MD, Anandasabapathy N, Liu Y, Altorki NK, Lauer P, Levy O, Glickman MS, Kaye J, Betel D, Philip M, Schietinger A. TOX is a critical regulator of tumour-specific T cell differentiation. Nature. 2019, 571(7764):270-274.
[13] Blank CU, Haining WN, Held W, Hogan PG, Kallies A, Lugli E, Lynn RC, Philip M, Rao A, Restifo NP, Schietinger A, Schumacher TN, Schwartzberg PL, Sharpe AH, Speiser DE, Wherry EJ, Youngblood BA, Zehn D. Defining'T cell exhaustion'. Nat Rev Immunol. 2019, 19(11):665-674.
[14] Anstee QM, Reeves HL, Kotsiliti E, Govaere O, Heikenwalder M. From NASH to HCC: current concepts and future challenges. Nature Reviews Gastroenterology Hepatology. 2019 Jul;16(7):411-428.
[15] Baumann T, Dunkel A, Schmid C, Schmitt S, Hiltensperger M, Lohr K, Laketa V, Donakonda S, Ahting U, Lorenz-Depiereux B, Heil JE, Schredelseker J, Simeoni L, Fecher C, Körber N, Bauer T, Hüser N, Hartmann D, Laschinger M, Eyerich K, Eyerich S, Anton M, Streeter M, Wang T, Schraven B, Spiegel D, Assaad F, Misgeld T, Zischka H, Murray PJ, Heine A, Heikenwälder M, Korn T, Dawid C, Hofmann T, Knolle PA, Höchst B. Regulatory myeloid cells paralyze T cells through cell-cell transfer of the metabolite methylglyoxal. Nature Immunology. 2020 May;21(5):555-566.
[16] Malehmir M, Pfister D, Gallage S, Szydlowska M, Inverso D, Kotsiliti E, Leone V, Peiseler M, Surewaard BGJ, Rath D, Ali A, Wolf MJ, Drescher H, Healy ME, Dauch D, Kroy D, Krenkel O, Kohlhepp M, Engleitner T, Olkus A, Sijmonsma T, Volz J, Deppermann C, Stegner D, Helbling P, Nombela-Arrieta C, Rafiei A, Hinterleitner M, Rail M, Baku F, Borst O, Wilson CL, Leslie J, O'Connor T, Weston CJ, Adams DH, Sheriff L, Teijeiro A, Prinz M, Bogeska R, Anstee N, Bongers MN, Notohamiprodjo M, Geisler T, Withers DJ, Ware J, Mann DA, Augustin HG, Vegiopoulos A, Milsom MD, Rose AJ, Lalor PF, Llovet JM, Pinyol R, Tacke F, Rad R, Matter M, Djouder N, Kubes P, Knolle PA, Unger K, Zender L, Nieswandt B, Gawaz M, Weber A*, Heikenwalder M*. Platelet GPIbα is a mediator and potential interventional target for NASH and subsequent liver cancer. Nature Medicine 2019 Apr;25(4):641-655.

SEQUENCE LISTING

<110>  Deutsches Krebsforschungszentrum Stiftung des öffentlichen
       Rechts

<120>  INHIBITORS FOR USE IN TREATING LIVER DISORDERS

<130>  D31768EP

<160>  1

<170>  PatentIn version 3.5

<210>  1
<211>  670
<212>  PRT
<213>  Homo sapiens

<400>  1

## Claims

1. **An inhibitor of endoplasmic reticulum (ER) stress signalling for use in the treatment or prevention of a liver disease** in a subject, comprising administering the inhibitor to the subject and thereby reducing or inhibiting endoplasmic reticulum (ER) stress signalling in a cell of the subject.

2. The inhibitor for use of claim 1, wherein the inhibitor is an inhibitor of activating transcription factor 6 alpha (ATF6) which inhibits or reduces the expression, stability, organelle translocation (e.g. from the Golgi into the endoplasmic reticulum) activation and/or function of AFT6 or nuclear AFT6 (nAFT6).

3. The inhibitor for use of claim 1 or 2, wherein the inhibitor reduces the nuclear concentration of AFT6, such as reduces the translocation of AFT6 to the nucleus in the cell, preferably a translocation of Golgi located AFT6 to the nucleus.

4. The inhibitor for use of any one of claims 1 to 3, wherein the liver disease is associated with any one or a combination of the following: fatty liver, hepatocyte hyperproliferation (liver cancer), expression of pro-inflammatory or immune-suppressive chemokines, presence of immune-suppressive cells such as T regulatory cells (TREG), proinflammatory immune cells such as monocytes and Myeloid Derived Suppressor Cells (MDSC).

5. The inhibitor for use of any one of claims 1 to 4, wherein the liver disease is a fatty liver, a non-alcoholic fatty liver disease (NAFLD), non-alcoholic steatohepatitis (NASH), liver cirrhosis or hepatocellular carcinoma (HCC).

6. The inhibitor for use of any one of claims 1 to 5, which is an inhibitor of the proteolytic cleavage of a membrane bound AFT6, such as a protease inhibitor, for example an inhibitor of Site-2 protease (S2P).

7. The inhibitor for use of to any one of claims 1 to 6, wherein the treatment is a reduced, stalled, or reversed progression of the liver disease, such as a progression of NAFLD/NASH into liver cirrhosis, preferably a reduced, stalled, or reversed progression of NASH into hepatocellular carcinoma (HCC).

8. The inhibitor for use of to any one of claims 1 to 7, wherein the treatment is a prevention of HCC in a NASH-patient at risk to develop cirrhosis and/or HCC.

9. The inhibitor for use of to any one of claims 1 to 8, wherein the treatment is performed in a patient at risk of developing NASH, such as a diabetic patient, an obese patient, or a patient suffering from the metabolic syndrome or from another metabolic disorder.

10. **A pharmaceutical composition for use in the treatment of a liver disease** in a subject, comprising an inhibitor recited in any one of the preceding claims and a pharmaceutically acceptable carrier and/or excipient, wherein the treatment comprises an administration of the pharmaceutical composition to the subject.

11. **A method for determining whether a subject has, or is at risk of developing, a liver disease,** the method comprising the step of:
(a) detecting an applicable biomarker in a biological sample from said subject;
wherein the detection of the applicable biomarker in the sample indicates a phenotype or a risk of developing a phenotype that is associated with the development of the liver disease; and
wherein the applicable biomarker is one selected from the group consisting of:
- AFT6, in particular the presence (or an amount) of or expression and/or activity of AFT6, preferably of nuclear AFT6;
- ER stress or ER stress signaling.

12. The method of claim 11, wherein the biological sample comprise cells or tissue of the subject, or an extract of such cells or tissue, in particular where such cells are those (usually, typically; or in the case or a specific subject as suspected to be) involved with the liver disease (eg hepatocytes, or tumour cells such as cells of HCC).

13. **A method for identifying and/or characterizing a compound suitable for the treatment of a liver disease in a subject,** the method comprising the steps of
(i) Providing a hepatocyte,
(ii) Inducing in the hepatocyte ER stress or ER stress signaling,
(iii) Contacting the cell of (ii) with a candidate compound,
wherein a reduced ER stress or ER stress signaling in the cell compared to a control indicates that the compound is suitable for the treatment of the liver disease.

14. **A method for identifying and/or characterizing a compound suitable for a treatment of a liver disease in a subject,** the method comprising the steps of
(a) bringing into contact a first cell expressing AFT6 and the candidate compound; and
(b) determining:
(i) the expression, activity, function and/or stability of protein or mRNA of AFT6, in particular, of proteolytically cleaved and/or nuclear AFT6, in the first cell; and
(ii) the ER stress signaling, or ER stress response in the first cell,
wherein:
(i) a reduced expression, activity function and/or stability of the AFT6, in said first cell contacted with the candidate compound compared to said first cell not contacted with said candidate compound; and
(ii) the ER stress signaling, or ER stress response in the first cell contacted with the candidate compound compared to ER stress signaling, or ER stress response of the first cell not contacted with the candidate compound;
indicates that the candidate compound is a compound suitable for the treatment of the liver disease.

15. **A kit for use** in a method of claim 13 or 14, the kit comprising a selection of compounds suspected to reduced ER stress or ER stress signaling, or reduce the expression, activity function and/or stability of the AFT6.
